# EUROPEAN PATENT APPLICATION

(11) **EP 3 967 291 A1**
(43) Date of publication of application: **16.03.2022**
(21) Application number: 20196309.7
(22) Date of filing: 15.09.2020
(51) Int. Cl.: A61K 8/06, A61K 8/41, A61Q 5/00, A61K 8/81, A61K 8/49, A61K 8/73, A61K 8/898, A61Q 5/02

(54) **HAIR COMPOSITION**

(71) Applicant: Unilever Global IP Ltd, Wirral, CH62 4ZD (GB)
(72) Inventor: AINGER, Nicholas John, Merseyside, CH63 3JW (GB); COLLINS, Luisa Zoe, Merseyside, CH63 3JW (GB); WOOD, Sally Elizabeth, Merseyside, CH63 3JW (GB)
(74) Representative: Tansley, Sally Elizabeth

(57) **Abstract**

A hair treatment composition comprising:
i) a cleansing phase comprising a surfactant;
ii) an oil-in-water emulsion comprising:
a) a silicone,
b) a piroctone compound, and
c) water; and

iii) a cationic deposition polymer.

## Description

### Field of the Invention

The present invention relates to a hair treatment composition, particularly an anti-dandruff shampoo composition, comprising an oil-in-water emulsion in which the emulsion comprises at least a piroctone compound and a silicone.

### Background of the Invention

Dandruff is a problem affecting many globally. The condition is manifested by the shedding of clumps of dead skin cells from the scalp. These are white in colour and provide an aesthetically displeasing appearance. A factor that contributes to dandruff are certain members of the *Malsassezia* yeasts. To combat these, hair treatment compositions are developed including various actives for their antidandruff effectiveness. Piroctone compound such as piroctone olamine is one such active.

A common problem with piroctone compound is that deposition of the active onto the desired surface during wash process is minimum. The desired surface is typically scalp and/or hair. For example, piroctone compound such as piroctone olamine is usually soluble in surfactants of the cleansing phase comprised in a hair treatment composition. During the excessive rinsing process, the majority of piroctone is likely to be washed away together with the surfactants. Poor deposition is correlated with low antidandruff activity, thus little mitigation of the ill-effects of dandruff. To date, there are attempts to offset this drawback by increasing the level of piroctone olamine in hair treatment composition. Such approach causes a variety of issues such as increased costs, potential instability of the formulation and potential adverse effect to hair sensory. Hence it is not an approach favoured by the industry.

DE102012203240A1 describes hair treatment composition with increased antidandruff effect and improved combing, gloss, elasticity of hair. The composition includes antidandruff agent selected from zinc pyrithione, climbazole, octopirox, ketoconazol, selenium disulphide, selenium containing vegetable oil, selenium containing plant extract, and a cationic amino silicone. Examples include shampoos and conditioners comprising Octopirox^{®} (Piroctone Olamine), amino silicone and surfactants.

US2013/0059929A1 describes a cosmetic or dermatological preparation, in which the preparation comprises at least one of benzethonium chloride, methylisothiazolinone, piroctone olamine, and lauroyl ethyl arginate, and does not contain a preservative that comprises a phenolic group. The preparations show improved sensory properties and sufficient microbiological stability.

US2002/0035161A1 describes a cosmetic/pharmaceutical oil-in-water emulsion including a discontinuous fatty phase dispersed in a continuous aqueous phase and comprising an effective amount of at least one biologically active agent (A) and an effect amount of an emulsifying system (B) therefor, said at least one biologically active (A) being non-solubilized therein in micronized particulate state, at least 80%, numerically, of said micronized particles having diameters ranging from 1 to 10 µm and at least 50%, also numerically, having diameters of less than 5 µm.

US2017/0165170A1 describes emulsions comprising at least three different preservatives. The cosmetic agents according to the invention are in the form of emulsions include synergistically effective preservative combinations. The cosmetic agents are used to clean and/or care for skin and/or hair.

Despite all the prior art, there remains a need to improve the deposition of piroctone compounds, especially piroctone acid or piroctone olamine, onto the surface of scalp and/or hair during washing process. There also remains a further need to improve the deposition onto said surface without adverse side effects to sensory, formulation rheology and conditioning performance.

The present invention addresses one or more needs described hereinbefore.

### Summary of the Invention

In a first aspect of the present invention, there is provided a hair treatment composition comprising: i) a cleansing phase comprising a cleansing surfactants; ii) an oil-in-water emulsion comprising (a) a silicone, (b) a piroctone compound, and (c) water; and iii) a cationic deposition polymer.

In a second aspect of the present invention, there is provided a method for making a hair treatment composition according to the first aspect of the invention, in which said method comprising steps of: mixing a piroctone compound, a silicone, and water to form an oil-in-water emulsion premix; adding the premix to a cleansing phase; and adding a cationic deposition polymer to the cleansing phase, in which the cationic polymer is added before, during or after the addition of premix.

In a third aspect of the present invention, there is provided a non-therapeutic method of treating a surface, comprising application of a hair treatment composition according to the first aspect of the invention, to a surface selected from hair and/or scalp.

Herein, 'antidandruff activity' should be understood to mean the effect of prevent, control, reduce or limit human dandruff.

Herein, 'sensory' should be understood to mean a physical condition of the hair treatment compositions and/or the scalp and hair stresses of heads perceived by consumers, e.g. appearance of the formulation, foaming, scalp/hair dryness, scalp irritation.

Herein, 'hair treatment composition' should be understood to mean a composition for typical application to hair and/or scalp of mammals, preferably humans. Such a composition may be in the form of a liquid, lotion, cream, foam, scrub, gel or bar. Non-limiting examples of such compositions include hair lotions, creams, shampoos, serums, or conditioners. Preferably, the hair treatment composition is an anti-dandruff hair shampoo.

It has been found that an oil-in-water emulsion comprising piroctone compound and silicone when included in a hair treatment composition can effectively deposit a piroctone compound onto the hair and/or scalp.

### Detailed Description of the Invention

Herein, any feature stated as 'preferred' in one aspect of the invention should be understood to be a preferred feature in the other aspect of the invention. Any feature described as 'preferred' should be understood to be particularly preferred in combination with a further preferred feature or features.

Herein, any feature of a particular embodiment of the present invention may be utilized in any other embodiment of the invention.

The word 'comprising' is intended to mean 'including' but not necessarily 'consisting of' or 'composed of'. The examples given in the description below are intended to clarify the invention but not to limit the invention. All weight percentages (wt%) are based upon the final weight of the composition unless indicated otherwise. Numerical ranges expressed in the format 'x-y' are understood to include x and y, unless specified otherwise. The numbers can be qualified by the term 'about'. When for a specific feature multiple preferred ranges are described in the format 'from x to y', it is understood that all ranges combining the different endpoints are also contemplated.

### Hair treatment composition

The composition preferably comprises a cosmetically acceptable carrier. By 'cosmetically acceptable carrier' is meant a carrier that is compatible with hair, skin, integuments or mucous membranes, not causing any unacceptable discomfort liable to discourage the consumer from using the composition. The preferred carrier is water.

The preferred format of the composition is an antidandruff shampoo. The anti-dandruff shampoo may suitably comprise from 50 to 90%, preferably from 60-80% water by weight of the total shampoo.

### Cleansing Phase

The cleaning phase comprises one or more cleansing surfactants. The cleansing surfactants refer to those which act to cleanse hair and/or scalp. The total level of cleansing surfactants is preferably from 0.5 to 45 %, more preferably from 1.5 to 20 %, most preferably from 5 to 15% by weight of the total composition.

Preferably, the cleansing surfactant comprises an anionic surfactant. Preferably, the anionic surfactant is alkyl sulphate and/or ethoxylated alkyl sulphate anionic surfactant. The preferred level of such surfactant is from 0.5 to 20% by weight of the total composition, more preferably from 5 to 15wt%.

Preferred alkyl sulphates are C₈₋₁₈ alkyl sulphate, more preferably C₁₂₋₁₈ alkyl sulphate, preferably in the form of a salt with a solubilising cation such as sodium, potassium, ammonium or substituted ammonium. Examples are sodium lauryl sulphate (SLS) or sodium dodecyl sulphate (SDS).

Preferred alkyl ether sulphates are those of formula (I): 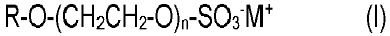 in which R is a straight or branched alkyl chain having 8 to 18 (preferably 12 to 18) carbon atoms; n is the average degree of ethoxylation and ranges from 0.5 to 3 (preferably from 1 to 3); and M is a solubilizing cation such as sodium, potassium, ammonium or substituted ammonium. An example is sodium lauryl ether sulphate (SLES). The most preferred example is SLES having an average degree of ethoxylate of from 0.5 to 3, preferably from 1 to 3.

The cleansing phase may comprise one or more further anionic surfactants which are cosmetically acceptable and suitable for topical application to hair and/or scalp. Examples of further anionic surfactants include alkyl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions according to the present invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate lauric monoglyceride sodium sulphate, sodium lauryl sulphate, sodium laureth sulphate, sodium cocyl sulphate, sodium cocoyl isethionate and mixtures thereof.

The composition can include co-surfactants, to help impart aesthetic, physical or cleansing properties to the composition. The co-surfactant is preferably comprised in the cleansing phase of the composition. An example of a co-surfactant is a nonionic surfactant, which can be included in an amount ranging from 0.5 to 10%, preferably from 2 to 8%, more preferably from 1 to 5% by weight of the total composition.

For example, representative nonionic surfactants that can be included in the treatment compositions, preferably shampoo compositions of the invention include condensation products of aliphatic (Cs - C₁₈) primary or secondary linear or branched chain alcohols or phenols with alkylene oxides, usually ethylene oxide and generally having from 6 to 30 ethylene oxide groups. Other representative nonionic surfactants include mono- or dialkyl alkanolamides. Examples include coco mono- or di-ethanolamide and coco monoisopropanolamide.

Further nonionic surfactants which can be included are the alkyl polyglycosides (APGs). Typically, the APG is one which comprises an alkyl group connected (optionally via a bridging group) to a block of one or more glycosyl groups. Preferred APGs are defined by the following formula (II): 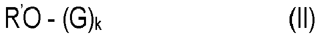 in which R' is a branched or straight chain alkyl group which may be saturated or unsaturated and G is a saccharide group. R' may represent a mean alkyl chain length of from about C₅ to about C₂₀. Preferably R' represents a mean alkyl chain length of from about C₈ to about C₁₂. Most preferably the value of R' lies between about 9.5 and about 10.5. G may be selected from C₅ or C₆ monosaccharide residues and is preferably a glucoside. G may be selected from the group comprising glucose, xylose, lactose, fructose, mannose and derivatives thereof. Preferably G is glucose. The degree of polymerisation, k, may have a value of from about 1 to about 10 or more; preferably, the value of k lies from about 1.1 to about 2; most preferably the value of m lies from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions (preferably shampoos) of the invention include the C₁₀-C₁₈ N-alkyl (Cₗ-C₆) polyhydroxy fatty acid amides, such as the C₁₂-C₁₈ N-methyl glucamides, as described for example in WO 92/06154 and US 5,194, 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10 wt.%, preferably from 2 to 8, more preferably from 1 to 5 % by weight of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, in which the alkyl and acyl groups have from 8 to 22 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is an amidobetaine amphoteric surfactant of general formula (III): 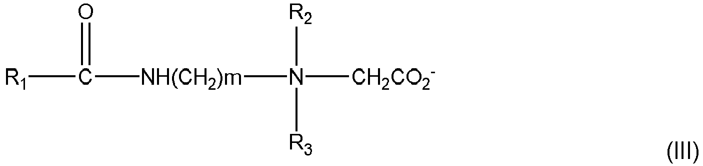 in which m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof. An example is cocamidopropyl betaine. The preferred level of such surfactant is from 0.5-10% by weight of the total composition, more preferably from 2-8 wt%, most preferably from 1-5 wt%.

A further optional but preferred surfactant is an alkyl glycinate and/or alkyl carboxyglycinate. If present, it is present at a level of from 1 to 8 wt.%, preferably 2 to 6 wt.%

Preferably the alkyl glycinate and/or alkyl carboxyglycinate has an alkyl group of from C₈₋₂₂ carbon atoms, in the form of a salt with a solubilising cation such as sodium, potassium, ammonium or substituted ammonium. Preferred glycinates are sodium coco glycinate and sodium cocoyl glycinate.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In preferred embodiments, the cleansing phase comprises an alkyl sulphate and/or ethoxylated alkyl sulphate anionic surfactant; and a betaine surfactant, preferably an alkyl amidopropyl betaine.

The total amount of surfactants (inclusive of any co-surfactants) in a hair treatment composition, is generally from 1 to 50 wt.%, preferably from 2 to 40 wt.%, more preferably from 10 to 25 wt.% by weight of the total composition.

### Oil-In-Water Emulsion

The oil-in-water emulsion is an essential feature of the present invention. The emulsion at least comprises a piroctone compound and a silicone.

The aqueous phase of the emulsion contains water. Suitably the emulsion comprises 25 to 85%, preferably from 40 to 70%, more preferably from 45 to 60% water by weight of the total emulsion.

The piroctone compound for use in the present invention may include piroctone acid, primary, secondary and tertiary olamine salts of piroctone acid (such as the diethanolamine and triethanolamine salts), and mixtures thereof, preferably piroctone acid, primary olamine salt of piroctone acid (i.e. piroctone olamine, also known as Octopirox^{®}) and mixtures thereof.

The piroctone compound useful in the present invention typically contains the structure defined by formula (IV): 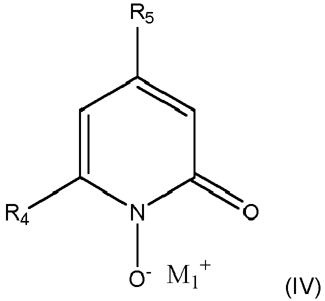 wherein R₄ is selected from C1-C17 hydrocarbon radicals, R₅ is selected from C1-4 alkyl, C2-4 alkenyl or alkynyl, hydrogen, phenyl or benzyl, and M₁ is selected from hydrogen, monoethanolamine (MEA), diethanolamine (DEA), or triethanolamine (TEA). Preferred R₄ group is (CH₃)₃CCH₂CH(CH₃)CH₂- and preferred R₅ is a methyl. More preferably, R₄ is (CH₃)₃CCH₂CH(CH₃)CH₂-, R₅ is a methyl and M₁ is a hydrogen or MEA. Most preferably, R₄ is (CH₃)₃CCH₂CH(CH₃)CH₂-, R₅ is a methyl and M₁ is hydrogen.

In the present invention, the piroctone compound is used in combination with a silicone in which the piroctone compound is preferably dispersible or soluble. It is highly preferable if within the dispersed phase of the emulsion the silicone and piroctone are within the same droplet.

In a one embodiment the piroctone compound is soluble in the silicone thus the piroctone is dissolved within the silicone droplet of the in the oil in water emulsion. By soluble it is meant that the solubility at 35°C is at least 2% w/w.

If the piroctone is dissolved within the silicone droplet of the in the oil in water emulsion; the silicone is present at a level of from 10 to 70% by weight of the total emulsion, preferably from 30 to 50 wt%;
If the piroctone is dissolved within the silicone droplet of the in the oil in water emulsion; the piroctone compound is preferably present at a level from 0.1 to 30% by weight of the total oil-in-water emulsion, preferably from 1 to 20 wt%, more preferably from 1 to 10wt%.

In an alternative preferred embodiment, the piroctone compound is dispersible in the silicone, thus the piroctone is present in the oil-in-water emulsion as a dispersion within a silicone droplet.

If piroctone is present in the oil in water emulsion as a dispersion within a silicone droplet.

The levels of piroctone compound in the oil-in-water emulsion is preferably from 1 to 40 wt% of the total emulsion, more preferably from 5 to 35 wt%, most preferably from 7 to 20 wt%

Silicone is present in the oil-in-water emulsion. The silicone is a conditioning agent intended to deposit onto hair remaining behind after rinsing of the hair with water. Typically, the piroctone compound comprises piroctone acid, piroctone olamine or mixtures thereof. The most preferred example is a piroctone acid.

The typical level of the piroctone compound is from 0.01 to 5% by weight of the total composition, preferably from 0.05 to 2 wt%, more preferably from 0.1 to 1.5 wt%.

The present invention has found that a piroctone compound comprised in an emulsion more readily deposits on the desired surface than piroctone comprised in the cleansing phase. To form an emulsion comprising piroctone compound, the piroctone compound is combined with a silicone and water to form an oil-in-water emulsion premix prior to its addition to the cleansing phase.

Preferably 50 wt% of the total level of the piroctone compound in the hair care composition is present, in the oil-in-water emulsion more preferably 75wt% of the total level of the piroctone compound is present, most preferably at least 95 wt% of the total level of the piroctone compound,

The techniques for analysis of formation of oil-in-water emulsion are known in the art. For example, microscopic analysis can be utilized to identify whether an emulsion phase has formed in a hair treatment composition comprising a cleansing phase. Such emulsion is usually identifiable as discrete or continuous 'phase' present in the cleansing phase. The cleansing phase is usually isotropic. The use of dyes may further aid in distinguishing the emulsion from other soluble phases dispersed in the hair treatment composition.

It is preferred if the dispersed phase of the water-in oil emulsion comprises silicone and piroctone within the same droplet.

The particle size of the silicone (D_{3,2}) droplet within the water in oil emulsion is preferably from 10nm to 10 microns, more preferably having a D_{3,2} mean droplet diameter from 50nm to 5 microns, most preferably from 100nm to 5 microns. D_{3,2} mean droplet diameter may be measured by means of a laser light scattering technique, for example using a 2600D Particle Sizer from Malvern Instruments.

The typical level of the silicone is from 0.1 to 17% by weight of the total composition, preferably from 0.5 to 13 wt%, more preferably from 1 to 10 wt%.

The weight ratio of the piroctone compound to silicone in the oil-in-water emulsion is suitable from 20:1 to 1:20, preferably from 1:15 to 15:1, more preferably from 1:10 to 10:1. The ratios are believed to be beneficial in terms of creating a stable oil-in-water emulsion.

Typical silicones may include polyalkyl siloxanes, polyaryl siloxanes, polyalkylaryl siloxanes, polyether siloxane copolymer and mixtures thereof.

The silicone may comprises a functionalized silicone. Suitable functionalized silicones include, for example, hydroxyl-, amino-, carboxy-, betaine-, quaternary ammonium-, carbohydrate-, hydroxy- and alkoxy-substituted silicones. The functionalized silicone may also contain multiple substitutions. Preferably, the functionalized silicone is an amino silicone, especially if the priroctone compound is to be solubilized in silicone.

Amino silicones are described in EP455185 and include trimethylsilylamodimethicone as depicted below, and are sufficiently water insoluble so as to be useful in the emulsion:

Si (CH₃)₃-O-[Si(CH₃)₂-O-]x-[Si(CH₃)(R₆-NH-CH₂CH₂-NH₂)-O-]y-Si(CH₃)₃

Wherein x+y is a number from about 50 to about 500, and the weight percent amine functionality is from about 0.03% to about 8%, and wherein R₆ is an alkylene group having from 2 to 5 carbon atoms. Preferably, the number x+y is from 100 to 300, and the weight percent amine functionality is from about 0.5% to 4%. As expressed herein, the weight percent amine functionality is measured by titrating a sample of the amino silicone against alcoholic hydrochloric acid to the bromocresol green end point. The weight percent amine is calculated using a molecular weight of 45 (corresponding to CH₃-CH₂-NH₂).

If the piroctone compound is present as a solution within the silicone of the water-in-oil emulsion amino silicones are preferred silicones.

By amino silicone is meant a silicone containing at least one primary, secondary or tertiary amine group, or a quaternary ammonium group. An example of a commercially available amino silicone useful in the emulsion of the invention is DC 2-8566 available from Dow Corning (INCI: dimethyl, methyl (aminoethylaminoisobutyl) siloxane). Examples of suitable amino silicones include polysiloxanes having the CTFA designation 'amodimethicone'. Specific examples of amino functional silicones suitable for use in the invention are the amino silicones DC 8220, DC8166, DC8466, and DC8950-114 (all ex Dow Corning) and GE 1149-75 (ex General Electric Silicones).

Suitable quaternary silicone polymers are described in EP0530974A. A preferred quaternary silicone is K3474 (ex Goldschmidt).

It is also possible to use a blend of amino and non-amino silicones.

It is preferable if at least 50 wt% of the total silicone in the composition is present in the emulsion, more preferably at least 75 wt%.

The viscosity of the silicone or silicone blend in isolation from the rest of the composition is suitably from 200-500,000 mm²/second at 25°C, preferably from 500-100,000, more preferably from 700-50,000, still more preferably from 1000-10,000, measured by Brookfield LVF, spindle3, 30rpm, 1 minute.

The oil-in-water emulsion may further comprise an emulsifier, preferably a non-ionic surfactant. The typical level of the emulsifier is from 0.01 to 5% by weight of the total composition, preferably from 0.05 to 2.5 wt%, more preferably from 0.1 to 1.5 wt%. The typical level of the emulsifier is from 0.1 to 20% by weight of the total emulsion, preferably from 0.5 to 10 wt%, more preferably from 1 to 7.5 wt%. Suitably, the weight ratio of the emulsifier to silicone is 1:1 to 1:50, preferably from 1:2 to 1:20, more preferably from 1:5 to 1:15.

The non-ionic surfactant suitable to act as emulsifier may include polyethylene glycol (PEG) compound, for example, trimethylnonylpolyethylene glycol (Trade name Tergitol TMN-6, ex Dow chemical or Sigma-Aldrich).

### Cationic deposition polymer

Cationic deposition polymer is an essential feature of the invention. The cationic polymer is often used in hair treatment composition for enhancing conditioning performance. In the present invention, it also leads to enhanced deposition of the piroctone compound.

Typically, the cationic deposition polymer is present at a level of from 0.01 to 5% by weight of the total composition, preferably from 0.05 to 2 wt%, more preferably from 0.1 to 1 wt%.

Suitable cationic deposition polymers include cationic polygalactomannans, which are polysaccharides composed principally of galactose and mannose units and are usually found in the endosperm of leguminous seeds, such as guar, locust bean, honey locust, flame tree, and the like. Guar flour is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is therefore one to two. Preferred cationic polygalactomannans are guar hydroxypropyltrimonium choride polymers.

Guar hydroxypropyltrimethylammonium chlorides for use in the invention are generally comprised of a nonionic guar gum backbone that is functionalized with ether-linked 2-hydroxypropyltrimethylammonium chloride groups and are typically prepared by the reaction of guar gum with N-(3-chloro-2-hydroxypropyl) trimethylammonium chloride.

Generally, the guar hydroxypropyltrimethylammonium chlorides have an average molecular weight (weight average molecular mass (Mw) determined by size exclusion chromatography) in the range 500,000 to 3 million g/mol, more preferably 800,000 to 2.5 million g/mol.

Generally, the guar hydroxypropyltrimethylammonium chlorides have a charge density ranging from 0.5 to 1.8 meq/g. The cationic charge density of the polymer is suitably determined via the Kjeldahl method as described in the US Pharmacopoeia under chemical tests for nitrogen determination. Preferred guar hydroxypropyltrimonium chlorides have a cationic charge density from 1.1 to 1.8 meq/g. Also suitable are mixtures of guars in which one has a cationic charge density from 0.5 to 1.1 meq/g, and one has a cationic charge density from 1.1 to 1.8 meq per gram.

Guar hydroxypropyltrimethylammonium chlorides for use in the invention are commercially available from Rhodia as JAGUAR ^{®} C13S, JAGUAR ^{®} C14 and JAGUAR ^{®} C17. Also preferred is boron-free crosslinked cationic guar hydroxypropyltrimethylammonium chloride, such as the guar polymer from Lambeti as Cesmetic ^{®} BF-7.

Other suitable cationic polymers for use in the present invention include polysaccharide polymers, such as cationic cellulose derivatives and cationic starch derivatives. Preferred cationic cellulose polymers are salts of hydroxyethyl cellulose reacted with trimethyl ammonium substituted epoxide, referred to in the industry (CTFA) as polyquaternium 10 and available from Dow Chemical as UCARE^{™} Polymer JR-30M. Other suitable types of cationic cellulose include the polymeric quaternary ammonium salts of hydroxyethyl cellulose reacted with lauryl dimethyl ammonium-substituted epoxide referred to in the industry (CTFA) a Polyquaternium 24.

Preferred for use with the invention are cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;

### Suspending Agent

Suspending agent is a preferred feature of the composition. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

A most preferred example is a crosslinked polyacrylate polymer.

Suspending agent, if included, will generally be present in a shampoo composition of the invention at levels of from 0.01 to 5 wt.%, preferably from 0.1 to 2.5 wt.%, more preferably from 0.25 to 1 wt.%.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

The pH of the composition of the invention preferably ranges from 3 to 9, more preferably from 4 to 8, most preferably from 4.5 to 6.5.

### Other optional components

The composition may optionally comprise one or more components for use in hair treatment products, provided that the optional components are physically and chemically compatible with the essential components described hereinbefore, and do not otherwise unduly impair sensory, formulation rheology and conditioning performance. Individual concentrations of such optional components may range from 0.001% to 10% by weight of the total composition, preferably from 0.01% to 5%wt%. Such components may include fragrance, dyes, and pigments, pH adjusting agents, pearlescers or opacifiers, viscosity modifiers, preservatives, and natural hair nutrients such as botanicals, fruit extracts, sugar derivatives and amino acids. The composition may comprise an additional silicone which is not in the oil-in-water emulsion phase. The additional silicone may be the same or different from the silicone comprised in the emulsion phase. The composition may also comprise additional antidandruff and/or anti-microbial agents such as pyridinethione salts, pine tar, sulfur, salicylic acid, azoles, selenium sulfide, or mixtures thereof.

### Method of use

The composition is used in a manner for treating a surface. An effective amount of the composition is applied to a desired surface selected from hair and/or scalp, that has been preferably wetted with water. The composition may be allowed to stay on the surface for a given time for it to take effect, preferably combined with massaging, before being rinsed off with water. The given time is preferably from 20 seconds to 2 minutes, more preferably from 30 seconds to 1 minute. The effective amount typically ranges from 1 g to 20g, preferably from 2.5 g to 10g.

### Method of making

A piroctone compound, a silicone and water are mixed to form an oil-in-water emulsion premix. Generally, the premix is formed by heating at least one of its components to a temperature from 50 to 100°C, more preferably from 60 to 70°C. The premix is added to a cleansing phase comprising at least one cleansing surfactant. A cationic deposition polymer is also added to the cleansing phase. The polymer can be added before, during or after the introduction of the premix. An optional ingredient can be added to the cleansing phase at any stage of the manufacture. It has been discovered that the piroctone compound must be combined with the silicone and water to form an oil-in-water emulsion prior to its addition to the cleansing phase, otherwise the deposition of the compound is poor.

The present invention may be illustrated by the following non-limiting examples.

### EXAMPLES

### Example 1

**Table 1 An oil-in-water emulsion (A)**

| **INCI name** | **Tradename** | **wt%** |
|---|---|---|
| Piroctone acid | Piroctone acid | 5 |
| Amodimethicone | DC 2-8566 | 44 |
| Trimethylnonylpolyethylene glycol | Tergitol TMN-6 | 5.2 |
| Water | water | To 100% |

Piroctone acid was dissolved in DC2-8566. The mixture was heated to 60-70°C. Mixing was applied to facilitate the dissolution. The water and Tergitol mixture was added stepwise to the silicone-Piroctone mixture until a thickening happened. This indicated the phase inversion which was also the formation of oil-in-water emulsion. Once all ingredients were added, the final mixture was allowed to cool to 20-30°C.

**Table 2 - Anti-dandruff shampoo compositions**

| **INCI name** | **Tradename** | **Composition 1(wt%)** |
|---|---|---|
| Sodium laureth sulphate (2EO) | Texapon N70 | 13 |
| Cocamidopropyl betaine | Tego Betain CK KB5 | 1 |
| Polyquaternium-10 | UCARE JR 30M | - |
| Guar Hydroxypropyltrimonium Chloride | Cesmetic BF-7 | 0.5 |
| Oil-in-water emulsion(A) | Oil-in-water emulsion(A) | 15 |
| Water and minors | | To 100% |

The composition was made by mixing the surfactant Texapon with a portion of water (39 wt%), followed by addition of the pre-mix A, the cationic polymer JR 30M, cosurfactant Tego Betain,and minors. The reminding water was added, and the final mixture was thoroughly blended to form an anti-dandruff shampoo.

## Claims

1. A hair treatment composition comprising:
i) a cleansing phase comprising a surfactant;
ii) an oil-in-water emulsion comprising:
a) a silicone,
b) a piroctone compound, and
c) water; and
iii) a cationic deposition polymer.

2. A hair treatment composition according to claim 1 in which the dispersed phase comprises silicone and piroctone within the same droplet.

3. A hair treatment composition according to any preceding claim in which silicone droplets within the emulsion have a D_{3,2} mean droplet diameter from 10nm to 10 microns, preferably from 50nm to 5 microns, more preferably from 100nm to 2 microns.

4. A hair treatment composition according to any preceding claim, in which the cleansing surfactant comprises alkyl sulphate and/or ethoxylated alkyl sulphate anionic surfactant; said surfactant is present at a level of from 0.5 to 20% by weight of the total composition, preferably from 5 to 15wt%.

5. A hair treatment composition according to any preceding claim, in which the oil-in-water emulsion (ii) further comprises an emulsifier, preferably a non-ionic surfactant.

6. A hair treatment composition according to any preceding claims, in which the cationic deposition polymer is a guar hydroxypropyltrimonium choride polymer.

7. A hair treatment composition according to any preceding claim, in which the silicone in oil-in-water emulsion (ii) is present at a level of from 0.1 to 17 % by weight of the total composition, preferably from 0.5 to 13 wt%, more preferably from 1 to 10 wt%.

8. A hair treatment composition according to any preceding claim in which at least 50 wt% of the total level of the piroctone compound in the composition is present in the oil-in-water emulsion, preferably 75wt% of the total level of the piroctone compound.

9. A hair treatment composition according to any preceding claim, in which the piroctone compound is present at a level of from 0.01 to 5% by weight of the total composition, preferably from 0.05 to 2 wt%, more preferably from 0.1 to 1.5 wt%.

10. A hair treatment composition according to any preceding claim, in which the cationic deposition polymer is present at a level of from 0.01 to 5% by weight of the total composition, preferably from 0.05 to 2 wt%, more preferably from 0.1 to 1 wt%.

11. A hair treatment composition according to any preceding claim in which the piroctone is present in the oil in water emulsion as a dispersion within a silicone droplet.

12. A hair treatment composition according to claim 11, in which the oil-in-water emulsion (ii) comprises silicone at a level from 10 to 40% by weight of the total emulsion, preferably from 15 to 35 wt%, more preferably from 20 to 30 wt%

13. A hair treatment composition according to claim 11 or 12, in which the oil-in-water emulsion (ii) comprises a piroctone compound at a level from 1 to 40 wt% of the total emulsion, preferably from 5 to 35 wt%, most preferably from 7 to 20 wt%.

14. A hair treatment composition according to any preceding claim in which the piroctone is dissolved within the silicone droplet of the in the oil in water emulsion.

15. A hair treatment composition according to claim 14, in which the oil-in-water emulsion (ii) comprises silicone at a level of from 10 to 70% by weight of the total emulsion, preferably from 30 to 50 wt%;

16. A hair treatment composition according to claim 14 or claim 15, in which the oil-in-water emulsion (ii) comprises a piroctone compound at a level from 0.1 to 30% by weight of the total emulsion, preferably from 1 to 20 wt%, more preferably from 1 to 10wt%.

17. A hair treatment composition according to any of claims 14 to 16 in which silicone is amino silicone

18. A hair treatment composition according to any of claims 14 to 17 in which the piroctone compound is piroctone acid.

19. A method for making a hair treatment composition according to any of the preceding claims, in which said method comprising steps of:
- mixing a piroctone compound, a silicone, and water to form an oil-in-water emulsion premix;
- adding the premix to a cleansing phase; and
- adding a cationic deposition polymer to the cleansing phase, in which the cationic polymer is added before, during or after the addition of premix.

20. A method according to claim 19, in which the premix is formed by heating at least one constituent of the premix to a temperature from 50 to100 °C, preferably from 60 to 70 °C.

21. A non-therapeutic method of treating a surface, comprising application of a hair treatment composition according to any of claims 1 to 18, to a surface selected from hair and/or scalp.
